# EUROPÄISCHE PATENTSCHRIFT

(11) **EP 4 477 195 B1**
(45) Veröffentlichungstag und Bekanntmachung des Hinweises auf die Patenterteilung: **21.01.2026**
(21) Anmeldenummer: 24181313.8
(22) Anmeldetag: 11.06.2024
(51) Int. Cl.: A61F 2/00, A61F 6/08

(54) **VAGINALPESSAR**
VAGINAL PESSARY
PESSAIRE VAGINAL

(30) Priorität: 12.06.2023 DE 102023115232; 24.11.2023 DE 102023132833
(43) Veröffentlichungstag der Anmeldung: 18.12.2024
(73) Patentinhaber: Arabin, Birgit, 14193 Berlin (DE)
(72) Erfinder: Arabin, Birgit, 14193 Berlin (DE)
(74) Vertreter: Brötz, Helmut

(56) Entgegenhaltungen:
- DE-U1- 202012 001 380
- GB-A- 670 349
- NL-B1- 2 025 355
- US-A- 4 858 624
- US-A1- 2019 008 674

## Beschreibung

Die vorliegende Erfindung betrifft ein Vaginalpessar, mit einem umlaufend geschlossenen Grundkörper, wobei der Grundkörper zwei (laterale) Seitenwandabschnitte, einen vorderen (anterioren) Wandabschnitt und einen hinteren (posterioren) Wandabschnitt aufweist und wobei eine mittlere Öffnung des Grundkörpers durch eine geschlossene, innere Konturlinie der Wandabschnitte begrenzt wird, gemäß dem Oberbegriff von Anspruch 1. Insbesondere bilden die Seitenwandabschnitte zusammen mit dem vorderen Wandabschnitt und dem hinteren Wandabschnitt eine umlaufende, das heißt in sich geschlossene, Seitenwandung des Pessars aus.

Vaginalpessare der in Rede stehenden Art werden zur Therapie gynäkologischer Krankheitsbilder eingesetzt, die mit einer Schwächung des Halteapparates der Gebärmutter bzw. der inneren, an den Vaginalbereich angrenzenden Organe zusammenhängen. Das Krankheitsbild betrifft ca. ein Drittel aller Frauen und tritt je nach Konstitution nach Schwangerschaften und Geburten durch die damit verbundenen Belastungen auf. Dadurch entstehen Versorgungslücken und hohe Kosten. Pessare stellen daher eine minimalinvasive, kostengünstige Alternative zu einer risikoreichen Operation dar. Inzwischen werden sie in den meisten Ländern sogar als erste Wahl vor einer Operation empfohlen (1-4).

Randomisierte Studien haben gezeigt, dass urogynäkologische Pessare Senkungsbeschwerden ebenso gut behandeln können, wie Operationen und zu hoher Patientenzufriedenheit beitragen können (5).

Die bisherigen Modelle dienten vor allem älteren Patientinnen zur Behandlung von Senkungsbeschwerden und/oder Inkontinenz als Folge von Schwangerschaft und Geburt.

In der Geburtsmedizin werden andere spezifische Pessare zur Verhinderung einer Frühgeburt bei Einlings- und Mehrlingsschwangerschaften eingesetzt. Dies sind Pessare, die um die Zervix gelegt werden, die Gebärmutter sakralwärts verschieben und den Druck auf das untere Uterinsegment reduzieren. Diese stehen hier nicht zur Debatte.

Die Therapie urogynäkologischer Beschwerden (Senkungen und/oder Inkontinenz) mit Vaginalpessaren erfolgt meist mit ringförmigen Modellen wie Ring-, Urethra- oder Siebschalenpessaren sowie mit Würfel-Pessaren, die sich durch ihre konkaven Wandstrukturen an die Scheidenwand anschmiegen. Generell sind zur Ausübung einer ausreichenden Stützfunktion von Senkungen innerer Organe oder der Urethra eine zuverlässige Lagefixierung der Pessare im Inneren der Vagina von großer Bedeutung.

Bisher verwendete ringförmige Pessare sind dabei davon abhängig, dass der Beckenboden noch stabil genug ist, um das Pessar abzustützen. Sie haben den Nachteil, dass die Vagina nicht kreisförmig, sondern eher oval ist und dadurch die lateralen Seitenwände der Vagina durch das Pessar weiter dilatiert werden, was sich ungünstig auf das Krankheitsbild auswirken kann.

Die Fixierung eines bisher gebrauchten Würfelpessars beruht auf den konkaven Seitenwänden, die sich mit ihrer Oberfläche an die Schleimhaut anlegen und das Pessar so fixieren können. Da diese Pessare jedoch den gesamten Raum der Vagina ausfüllen, muss man sie jeden Abend entfernen und am Tag wiedereinsetzen. Dies ist vielen Patientinnen lästig. Zusätzlich haben die Würfel notgedrungen einen Faden zur erforderlichen Handhabung, der Patientinnen auf Dauer auch stören kann. Insofern könnten die bisher aus der Praxis und dem Stand der Technik bekannten Vaginalpessare verbessert werden. Dazu kommt, dass die Fixierung der ringförmigen Pessare auf Kosten der Dilatation und die der Würfelpessare auf Kosten eines möglicherweise am Abfluss gehinderten Ausflusses (z.B. bei Menstruation) in ihrer therapeutischen Behandlungsmöglichkeit eingeschränkt sind.

Durch neuere Ultraschalluntersuchungen im 3D Verfahren wurden auch die Ursachen von Senkungsbeschwerden spezifischer beschrieben: Oft kommt es zu einem Abriss des Beckenbodenmuskels "Levator ani" von der Beckenwand (6). Dann hilft auch keine Beckenbodentherapie mehr (7).

Den oben beschriebenen Erkenntnissen wurde diese neue Entwicklung angepasst. Die erhöhten Seitenwände üben eine Art "Massagefunktion" auf die lateralen Vaginalwände aus und führen im Ultraschall wieder zu Kontraktionen selbst abgerissener Muskelfasern.

Die DE 20 2012 001380 U1 betrifft ein Stützpessar zur Prophylaxe und Therapie einer drohenden Frühgeburt nach Hamann Bernd vom 15.2.2012.In der US 4,858,624 ist ein Verhütungspessar beschrieben.

Aus der NL 2025 355 B1 ist ein Pessar bekannt, das einen tassenartigen Grundkörper aufweist.

Die GB 670349 A beschreibt ein metallisches Pessar mit elliptischer Form und offenbart den Oberbegriff des Anspruchs 1.

Aufgabe der vorliegenden Erfindung ist es daher, einen Prototyp eines Vaginalpessars zur Verfügung zu stellen, das eine nebenwirkungsfreie zuverlässige Behandlung im Rahmen der Prävention und Behandlung von Senkungen innerer Organe und/oder Belastungsinkontinenz zulässt.

Die genannte Aufgabe wird durch ein Vaginalpessar mit den Merkmalen von Anspruch 1 gelöst. Vorteilhafte Weiterentwicklungen sind Gegenstand der Unteransprüche.

Bei dem erfindungsgemäßen Vaginalpessar ist vorgesehen, dass die innere Konturlinie der Wandabschnitte eine im Wesentlichen eiförmige und von der Kreisform abweichende mittlere Öffnung begrenzt. Dies entspricht eher der eigentlichen Anatomie und soll die Dilatation der lateralen Vaginalwände abschwächen. Durch die konkaven nach innen gestülpten Flächen der Seitenwände soll nicht nur die Haltefunktion des Pessars verbessert werden, sondern auch eine Zusammenführung der lateralen Wände erfolgen, da die eiförmige Kontur den anatomischen Bedingungen im eingesetzten Zustand im Vaginalbereich optimal angepasst ist und ohne laterale Dehnung an der umgebenden Gewebestruktur anliegt. Durch die Beschaffenheit der Außenwände übernimmt das erfindungsgemäße Vaginalpessar eine zuverlässige bzw. widerstandsfähige Stützfunktion. Darüber hinaus weist das erfindungsgemäße Vaginalpessar auch einen hohen Widerstand gegen ein Kollabieren bzw. radial nach innen gerichtete Verformungen auf, was eine effiziente Stützfunktion weiterhin zulässt, da stets eine ausreichend hohe radial äußere Abdichtung sichergestellt ist. Zusätzlich erlaubt es im Gegensatz zum Würfelpessar das Ablaufen von Fluor oder Blut.

Der Begriff "eiförmig" im Sinne der Erfindung bezieht sich auf die Geometrie einer inneren Konturlinie einer umlaufenden, die mittlere Öffnung des Pessars begrenzenden Seitenwandung des erfindungsgemäßen Pessars, mit Bezug auf eine Draufsicht auf das Pessar.

Vorzugsweise weist die Öffnung abgerundete Ecken auf. Die Öffnung kann vorzugsweise ein spitzes Ende im Bereich eines im eingesetzten Zustand des Pessars vorderen Wandabschnitts des Pessars und ein stumpfes Ende im Bereich eines hinteren Wandabschnitts aufweisen.

Der Begriff "eiförmig" im Sinne der Erfindung ist breit zu verstehen.

Der Begriff "eiförmig" bzw. "oval" im Sinne der Erfindung bezeichnet eine ebene, rundliche, konvexe Figur bzw. Konturlinie der mittleren Öffnung des erfindungsgemäßen Pessars in einer Ebene bzw. in der Draufsicht des Pessars, die im weitesten Sinne dem Profil eines Vogeleis ähnelt. Sie umfasst erfindungsgemäß allerdings keine Kreisform, aber gegebenenfalls eine Ellipse als Spezialfall.

Eine eiförmige bzw. ovale, nicht-kreisförmige mittlere Öffnung im Sinne der Erfindung besitzt insbesondere die folgenden Eigenschaften: Die Konturlinie der mittleren Öffnung besitzt insbesondere keine Schlingen oder Schlaufen und/ oder die orientierte Krümmung der Konturlinie besitzt insbesondere keinen Vorzeichenwechsel, d. h., je nach Durchlaufsinn ist die orientierte Krümmung für jeden Punkt der Konturlinie entweder nichtnegativ oder nichtpositiv, was bedeutet, dass sie keine Wendungen oder Einbuchtungen besitzt. Man kann die Konturlinie nur in einer reinen Linkskurve bzw. Rechtskurve durchlaufen.

Die Krümmung der Konturlinie der mittleren Öffnung des erfindungsgemäßen Pessars kann auf keinem Abschnitt verschwinden.

Es ist aber vorzugsweise so, dass sich die Konturlinie der mittleren Öffnung auch aus Bögen, insbesondere Kreisbögen, und wenigstens einem im Wesentlichen geraden Konturlinienabschnitt oder einem bogenförmigen Konturlinienabschnitt mit sehr geringer Krümmung zusammensetzt.

Besonders bevorzugt setzt sich die Konturlinie zusammen aus einem vorderen, konvexen Konturlinienabschnitt und einem hinteren, ebenfalls konvexen oder vorzugsweise im Wesentlichen geradlinigen Konturlinienabschnitt. Vorzugsweise ist der vordere, konvexe Konturlinienabschnitt stärker gekrümmt bzw. spitzer als der hintere, konvexe Konturlinienabschnitt, der dann als "stumpf" bezeichnet werden kann.

Besonders bevorzugt kann die Konturlinie einen hinteren, geradlinigen Konturlinienabschnitt aufweisen, der über zwei seitliche konvexe Konturlinienabschnitte mit dem vorderen, konvexen Konturlinienabschnitt verbunden ist. Der vordere, konvexe Konturlinienabschnitt kann stärker gekrümmt sein als die beiden seitlichen Konturlinienabschnitte. Die Konturlinie verläuft insbesondere an den Übergängen der Konturlinienabschnitte gerundet. Die Konturlinie der mittleren Öffnung kann eine Symmetrieachse besitzen. Dies ist aber nicht zwingend notwendig. Eine eiförmige innere Konturlinie der mittleren Öffnung des Pessars erfordert nicht zwingend eine symmetrische Ausbildung bzw. eine diesbezügliche Symmetrieachse. Auch asymmetrisch ausgebildete Konturlinien sind durch die erfindungsgemäße Lehre erfasst, falls dies die Behandlung verbessert. Gleichwohl ist eine symmetrische, eiförmige Ausbildung der inneren Konturlinie bevorzugt, um sich im Einsatzzustand den anatomischen Bedingungen besonders zuverlässig abdichtend anpassen zu können. Auch aus Handhabungsgründen einer routineartigen Herstellung hat sich eine symmetrische Ausbildung der inneren Konturlinie als vorteilhaft erwiesen.

Mit anderen Worten ist die eiförmige innere Konturlinie, insbesondere im Gegensatz zu einer kreisförmigen Konturlinie, besser an die Anatomie der Vagina angepasst. Dadurch wird das Einführen sowie das Tragegefühl des eingesetzten Vaginalpessars verbessert.

Auch eine trapezförmige innere Konturlinie, vorzugsweise mit abgerundeten Eckbereichen, kann erfindungsgemäß noch als eiförmige Konturlinie bezeichnet werden.

Besonders bevorzugt weist die innere Konturlinie einen zumindest im wesentlichen eiförmigen Verlauf mit einem im Wesentlichen spitzen Ende auf der Seite des vorderen (anterioren) Wandabschnitts und mit einem im Wesentlichen stumpfen Ende auf der Seite des hinteren (posterioren) Wandabschnittes auf. Insofern bildet der eiförmige Verlauf eine bevorzugte Ausführungsform der allgemein ovalförmigen Öffnung des erfindungsgemäßen Vaginalpessars.

Der elliptische Verlauf stellt eine bevorzugte Ausführung der Öffnung dar, die durch die innere Konturlinie begrenzt ist. Die Ellipsen-Form ist mathematisch definiert und insbesondere in Form eines Kegelschnitts ausgebildet. Insbesondere ist demgemäß vorzugsweise vorgesehen, dass der vordere (anteriore) Wandabschnitt eine geringere Erstreckung in Umgangsrichtung aufweist als der hintere (posteriore) Wandabschnitt. Dies führt dazu, dass die Konturlinie am vorderen Ende spitzer und am hinteren Ende stumpfer verläuft. Dies ermöglicht eine besonders präzise bzw. zuverlässige Abstimmung an die anatomischen Begebenheiten im eingesetzten Zustand des Vaginalpessars. Sollte jedoch das Krankheitsbild vorwiegend durch einen anterioren Defekt gekennzeichnet sein, kann man das Pessar auch mit der weiteren Seite nach vorn einsetzen.

Gemäß einer besonders bevorzugten Ausführungsform der vorliegenden Erfindung ist der vordere (anteriore) Wandabschnitt konvex gebogen und/oder der hintere (posteriore) Wandabschnitt des erfindungsgemäßen Pessars im Wesentlichen konvex gebogen oder im Wesentlichen geradlinig verlaufend ausgebildet. Dies gestattet eine besonders schonende und gleichzeitig zuverlässige Anbindung des Vaginalpessars.

Vorzugsweise ist zudem vorgesehen, dass der vordere (anteriore) Wandabschnitt und/oder der hintere (posteriore) Wandabschnitt in einer Draufsicht auf die Öffnung im Wesentlichen unterschiedlich konvex gebogen ist bzw. sind. Dabei ist der anteriore Wandabschnitt vorzugsweise stärker gebogen als der hintere Wandabschnitt, um eine spitzere bzw. kürzere Ausbildung des anterioren Wandabschnitts gegenüber dem hinteren stumpfen bzw. längeren Wandabschnitt umzusetzen.

Gemäß einer bevorzugten Ausführungsform ist die innere Konturlinie der mittleren Öffnung spiegelsymmetrisch zu einer Mittellängsachse und/oder Mittelquerachse des Grundkörpers ausgebildet. Die Spiegelsymmetrie kann sich dabei relativ zu einer einzigen Mittelachse beziehen, beispielsweise zu einer Mittellängsachse im Fall einer eiförmigen oder trapezförmigen Konturlinie. Es ist jedoch auch eine spiegelsymmetrische Ausbildung der Konturlinie zur Mittellängsachse und zu einer Mittelquerachse möglich, beispielsweise bei einer elliptischen Ausbildung der inneren Konturlinie bzw. der Öffnung.

Besonders bevorzugt weist die innere Konturlinie entlang der mittleren Öffnung einen durchgehend konvexen Verlauf auf, der im Bereich des vorderen (anterioren) Wandabschnitts stärker gekrümmt ist als im Bereich des hinteren (posterioren) Wandabschnitts. Im Bereich der Seitenwandabschnitte ist die innere Konturlinie insbesondere gleich gekrümmt, wohingegen an den posterioren und anterioren Wandabschnitten eine unterschiedliche Krümmung der inneren Konturlinie vorliegt.

Gemäß einer bevorzugten Ausführungsform ist vorgesehen, dass die Dicke der Wandabschnitte, vorzugsweise der lateralen Seitenwandabschnitte, ausgehend von einer proximalen Außenseite des Grundkörpers in Richtung zu einer distalen Außenseite des Grundkörpers abnimmt. Mit anderen Worten ausgedrückt nimmt die Wanddicke bezogen auf den Einsetzzustand des Pessars im Körper in Richtung zur Körpermitte hin zu. Alternativ oder zusätzlich sind die Wandabschnitte, vorzugsweise die Seitenwandabschnitte, zur distalen Außenseite des Grundkörpers hin zulaufend und/oder verjüngend ausgebildet.

Beispielsweise sind die Wandabschnitte, insbesondere die Seitenwandabschnitte, in einem Querschnitt senkrecht zur Öffnung bzw. zum Grundkörper tropfen- und/oder eiförmig ausgebildet.

Die Seitenwandabschnitte sind laschenförmig ausgebildet. Dies erleichtert auch als Nebeneffekt das Einführen in den Vaginalbereich.

Gemäß einer nicht beanspruchten Ausführungsform begrenzen die Wandabschnitte des erfindungsgemäßen Vaginalpessars eine im Wesentlichen trapezoide Öffnung. Dadurch lassen sich ebenfalls entsprechende Vorteile realisieren. Eine innere Konturlinie zur Ausbildung einer trapezoiden Öffnung ist ebenfalls breit zu verstehen und erfordert nicht zwingend gerade Wandabschnitte zur Ausbildung der inneren Konturlinie. So können auch bogenförmige bzw. konvexe Wandabschnitte, insbesondere im Bereich der Seitenwandabschnitte, eine trapezoide innere Konturlinie bilden.

Der Grundkörper kann eine sich in Umfangsrichtung vorzugsweise stetig ändernde Höhe der umlaufenden Seitenwandung aufweisen, wobei, vorzugsweise, eine kleinere Höhe am anterioren Wandabschnitt und/oder eine größere Höhe am posterioren Wandabschnitt erreicht werden kann. Die Höhe am anterioren Wandabschnitt und die Höhe am posterioren Wandabschnitt können auch im Wesentlichen gleich sein. Die Seitenabschnitte weisen vorzugsweise die größte Höhe auf, weiter vorzugsweise in einem mittleren Bereich der Seitenwandabschnitte, wobei die Höhe von einer Maximalhöhe auf die Höhe am anterioren Wandabschnitt und/oder die Höhe am posterioren Wandabschnitt abfällt. Der Begriff "Höhe" im Sinne der Erfindung beschreibt die Erstreckung bzw. die Ausdehnung der Wandabschnitte zwischen einer distalen Außenfläche und einer proximalen Außenfläche der Wandabschnitte.

Die Änderung der Höhe in Umfangsrichtung des Grundkörpers erfolgt erfindungsgemäß vorzugsweise stetig und/oder kontinuierlich. Eine minimale Höhe kann dabei am anterioren Wandabschnitt und eine maximale Höhe am posterioren Wandabschnitt erreicht werden. Es ist jedoch auch möglich, dass die maximale Höhe an einem Seitenwandabschnitt erreicht wird, insbesondere wobei die maximale Höhe an beiden gegenüberliegenden Seitenwandabschnitten erreicht wird.

Die sich ändernde Höhe in Umfangsrichtung des Grundkörpers ist mit einer verbesserten Stabilität des Vaginalpessars mit angrenzenden Gewebeabschnitten verbunden. Die sich in Umfangsrichtung ändernde Höhe ermöglicht eine flexible Anpassung an die anatomischen Bedingungen der Vagina, wobei stets eine zuverlässige Anlage des Vaginalpessars in Umfangsrichtung an die angrenzenden Gewebeabschnitte gewährleistet ist.

Besonders bevorzugt wird die gleiche minimale Höhe am anterioren Wandabschnitt und am posterioren Wandabschnitt erreicht, insbesondere wobei die maximale Höhe an beiden Seitenwandabschnitten erreicht wird. Die proximalen Außenflächen der Seitenwandabschnitte, des vorderen Wandabschnitts und des hinteren Wandabschnitts des Pessars liegen vorzugsweise in einer Ebene. Auf der distalen Außenseite des Grundkörpers weist der Grundkörper dann eine sich in Umfangsrichtung ändernde Höhe auf.

Erfindungsgemäß ist bei einem Vaginalpessar der eingangs genannten Art zur Lösung der in Rede stehenden Aufgabe vorgesehen, dass der Grundkörper an einem Seitenwandabschnitt, jeweils eine auf der Außenseite des Grundkörpers geöffnete, taschenartige Vertiefung aufweist, um eine bessere Haftung für anliegendes Gewebe im Einsatzzustand bzw. im eingesetzten Zustand des Vaginalpessars zu bilden. Das Besondere dieser Ausführungsform ist, dass sich in beiden Seitenwandabschnitten eine oder mehrere, insbesondere in den Seitenwandabschnitten gegenüberliegende, Einbuchtungen bzw. taschenförmige Vertiefungen befinden, wodurch sich die Wände besser an die Vaginalwand anheften und dadurch für einen sicheren Halt sorgen.

Wesentliches Kennzeichen der vorliegenden Erfindung gemäß dieses Aspekts sind die Seitenwände, die in der Querschnittsansicht im Wesentlichen konvex nach innen gebogen bzw. gewölbt sind. Dies gestattet eine besonders schonende und gleichzeitig zuverlässige Anbindung des Vaginalpessars an die Vaginalschleimhaut, da sich ein partieller Vakuumeffekt aufbaut. Dadurch hält das Pessar bei der Verifizierung am Schweinemodell (dem Menschen ähnliche Vagina) bei Zug nach außen ca. 5mal so gut wie ein gewöhnliches Ringpessar (im Versuchsdurchschnitt bei geschlossenen Vertiefungen 18 Newton im Vergleich zu 3 Newton Zugkraft zur Entfernung erforderlich, siehe unten, Tabelle 1).

Der in Rede stehende Aspekt der wenigstens einen taschenförmigen Vertiefung in einem zugeordneten Wandabschnitt des Pessars hat sich im Hinblick auf den ersten Aspekt, wonach die innere Konturlinie der Wandabschnitte eine im Wesentlichen eiförmige und von der Kreisform abweichende Öffnung begrenzt, als besonders vorteilhaft erwiesen. Während ein Pessar mit eiförmig ausgebildeter innerer Konturlinie sich verbessert an die Anatomie im Inneren der Vagina anpasst, wird durch die wenigstens eine Einbuchtung bzw. taschenförmige Vertiefung gewährleistet, dass trotz der verbesserten Anpassbarkeit eine zuverlässige Anbindung des Vaginalpessars an die Vaginalschleimhaut gewährleistest ist. Insofern wird die sich anatomisch anpassende eiförmige Ausbildung des Vaginalpessars in zweckmäßiger Weise durch die Einbuchtung bzw. die taschenförmige Vertiefung ergänzt. Dadurch ergänzen sich die beiden positiven Wirkungen der in Rede stehenden beiden Aspekte, nämlich der eiförmigen Ausbildung einerseits sowie der taschenförmigen Vertiefung andererseits, in einer sich verstärkenden bzw. synergistischen Weise, wobei bei einem komfortablen bzw. angenehmen Tragegefühl gleichzeitig eine zuverlässige Fixierung des Vaginalpessars am Einsatzort bzw. im Einsatzzustand ermöglicht wird.

Besonders bevorzugt ähnelt die Geometrie des erfindungsgemäßen Pessars einem Ringpessar mit nicht-kreisförmigem Querschnitt, wobei, vorzugsweise, die lateralen Wandabschnitte eine im Vergleich zu dem posterioren und anterioren Wandabschnitt größere Höhe aufweisen und/oder laschenartig und/oder sich distal verjüngend ausgebildet sind, so dass beim Einsetzen des Pessars dieses an den lateralen Wandabschnitten ergriffen und gehalten werden kann. Die posterioren und anterioren Wandabschnitte können einen im Wesentlichen kreisförmigen Querschnitt aufweisen. Weiter vorzugsweise ist dann im mittleren Bereich des jeweiligen Seitenwandabschnitts, in dem der Seitenwandabschnitt eine maximale Höhe erreicht, wenigstens eine taschenartige Vertiefung vorgesehen, wobei das Einbringen der Vertiefung durch die sich distal verjüngende Wanddicke des Seitenwandabschnitts erleichtert wird.

Die in Rede stehende taschenförmige Vertiefung bzw. Einbuchtung fungiert somit als Aufnahmetasche und/oder Saugnapf, wobei im Rahmen des Einsetzens innerhalb der taschenförmigen Vertiefung bzw. Einbuchtung im Anlagezustand an das angrenzende Gewebe ein Unterdruck erzeugt wird, einhergehend mit einer besonders zuverlässigen und/oder resistenten Fixierung des Vaginalpessars am Einsatzort.

Die Vertiefung kann auf einer Innenseite des Grundkörpers vorzugsweise geschlossen ausgebildet sein und bildet dann eine innenseitig geschlossene und auf der Außenseite des Pessars geöffnete Tasche in dem Wandabschnitt des Grundkörpers aus. Vorzugsweise können alle Vertiefungen geschlossen ausgebildet sein. Bei mehreren Vertiefungen kann auch wenigstens eine Vertiefung geschlossen und wenigstens eine auf einer Innenseite des Grundkörpers geöffnet ausgebildet sein, wobei über eine geöffnete Vertiefung ein Ablauf von Körperflüssigkeiten erreicht werden kann. Es besteht aber auch die Möglichkeit, mehrere Vertiefungen auf der Innenseite des Grundkörpers geöffnet auszubilden, mit der Maßgabe, dass wenigstens eine Vertiefung geschlossen ausgebildet ist, insbesondere um die Saugwirkung zu gewährleisten.

Vorzugsweise ist die Vertiefung radial nach innen gerichtet, wobei die Vertiefung auf der bzw. den Innenseite(n) des Grundkörpers durch einen dünnwandigen Wandabschnitt gebildet wird, der gegenüber den an die Vertiefung angrenzenden Wandabschnitten des Grundkörpers nach innen ausgestülpt ist. Die Ausstülpung ermöglicht eine Verformbarkeit und soll zur Erhöhung bzw. Erzeugung der Saugwirkung innerhalb der taschenförmigen Vertiefung im Einsetzzustand dienen. Die Ausbildung einer taschenartigen Vertiefung kann zu einer lediglich vernachlässigbaren Verminderung der Steifigkeit des erfindungsgemäßen Vaginalpessars im Vergleich zu einem geometrisch gleichen Vaginalpessar ohne taschenartige Vertiefung führen.

Besonders bevorzugt führt die Ausbildung einer taschenartigen bzw. taschenförmigen Vertiefung zu einer lediglich vernachlässigbaren Verminderung der Steifigkeit des erfindungsgemäßen Vaginalpessars im Vergleich zu einem herkömmlichen Ringpessar als Referenzkörper, das insbesondere eine kreisförmige mittlere Öffnung aufweist und einen im Wesentlichen gleichen Durchmesser und/oder eine im Wesentlichen gleiche Höhe der die mittlere Öffnung begrenzenden Wandabschnitte des Ringpessars. Der Durchmesser, Umfang und/oder die Höhe des Ringpessars als Referenzkörper kann dabei vorzugsweise dem Durchmesser, Umfang und/oder der Höhe des anterioren und/oder posterioren Wandabschnitts des erfindungsgemäßen Pessars entsprechen.

Der Begriff "Steifigkeit" im Sinne der Erfindung betrifft insbesondere den Widerstand des Pessars gegen eine Krafteinwirkung von außen auf einen Wandabschnitt, insbesondere auf gegenüberliegende Wandabschnitte, weiter insbesondere auf beide Seitenwandabschnitte des Grundkörpers in Richtung zur Mitte des Vaginalpessars bzw. beim (seitlichen) Zusammendrücken des Vaginalpessars im Bereich der Wandabschnitte. Weiter insbesondere betrifft der Begriff "Steifigkeit" im Sinne der Erfindung den Widerstand des Pessars gegen Aufbringen einer Druckkraft auf die Wandabschnitte in proximaler Richtung unterhalb der wenigstens einen taschenartigen Vertiefung.

In Vergleichsberechnungen wurden Steifigkeiten gegen Verformung beim Aufbringen von Zug- und Druckkräften auf laterale Wandabschnitte eines erfindungsgemä-ßen Pessars unterhalb taschenförmiger Vertiefungen und eines Ringpessars als Referenzobjekt ermittelt. Insbesondere wurden Vergleichsberechnungen für ein erfindungsgemäßes Pessar durchgeführt, dessen Geometrie einem Ringpessar mit nicht-kreisförmigen Querschnitt ähnelt, wobei die lateralen Wandabschnitte eine im Vergleich zu dem posterioren und anterioren Wandabschnitt größere Höhe aufweisen und laschenartig und/oder sich distal verjüngend ausgebildet sind. Die posterioren und anterioren Wandabschnitte weisen einen im Wesentlichen kreisförmigen Querschnitt auf. Im mittleren Bereich des jeweiligen Seitenwandabschnitts erreicht der Seitenwandabschnitt eine maximale Höhe und es ist jeweils eine taschenartige Vertiefung vorgesehen. Der Durchmesser, Umfang und/oder die Höhe des Ringpessars als Referenzkörper entspricht dem Durchmesser, Umfang und der Höhe des anterioren und/oder posterioren Wandabschnitts des erfindungsgemäßen Pessars. Bei den Berechnungen konnte ermittelt werden, dass die Steifigkeit des erfindungsgemäßen Pessars zwischen 30% bis 100% der Steifigkeit des Ringpessars als Referenzobjekt beträgt.

Alternativ oder zusätzlich ist vorgesehen, dass die Vertiefung durch einen Wandabschnitt des Grundkörpers gebildet wird, der gegenüber an die Vertiefung angrenzenden Wandabschnitten des Grundkörpers eine höhere Elastizität und/oder eine geringere Wanddicke und/oder eine geringe Härte aufweist. Insbesondere eine verringerte Wanddicke ermöglicht eine verbesserte Verformbarkeit und/oder eine erhöhte Nachgiebigkeit des Pessars im Bereich der Vertiefung, um die Saugwirkung entsprechend zu verstärken und/ oder zu erzeugen.

Vorzugsweise wird die Vertiefung durch einen dünnwandigen Wandabschnitt des Grundkörpers gebildet, der gegenüber einem angrenzenden vertiefungsfreien Wandabschnitt des Grundkörpers eine um wenigstens 40 %, vorzugsweise wenigstens 60 %, besonders bevorzugt wenigstens 80 %, ganz besonders bevorzugt wenigstens 90 %, verringerte Materialstärke bzw. Wanddicke aufweist.

Alternativ oder zusätzlich weist der dünnwandige Wandabschnitt, insbesondere im Zentrum der Vertiefung, eine Dicke im Bereich von 0,1 mm bis 4 mm, vorzugsweise 0,5 bis 3 mm, besonders bevorzugt 0,5 bis 2 mm, auf.

Alternativ oder zusätzlich weist der an die Vertiefung angrenzende, nicht vertiefte, Wandabschnitt des Grundkörpers eine Dicke im Bereich von 3 bis 12 mm, vorzugsweise im Bereich von 5 bis 10 mm, besonders bevorzugt im Bereich von 6 bis 8 mm, auf.

Die Herstellung des Grundkörpers bzw. des Vaginalpessars kann prinzipiell durch unterschiedliche Herstellungs- bzw. Produktionsverfahren, beispielsweise auch durch Vakuum-, Hand-, Umform- oder Spritzguss erfolgen. Besonders bevorzugt erfolgt dabei die Formgebung zur Ausbildung der taschenartigen Vertiefung stets durch ein entsprechendes Werkzeug, unabhängig von dem gewählten Produktionsverfahren.

Prinzipiell sind alle für medizinische Anwendungen zugelassenen Kunststoffe bzw. Silikone, insbesondere Vergußmassen, Flüssig- oder Festsilikone, zur Herstellung des Grundkörpers einsetzbar.

Besonders bevorzugt ist der Grundkörper aus einem Silikonkunststoff hergestellt und/oder ausgebildet.

Alternativ oder zusätzlich ist der Grundkörper aus einem Kunststoffmaterial mit nachgewiesener Biokompatibilität nach DIN EN ISO 10993 hergestellt.

Die Härte des eingesetzten Materials kann dabei je nach Formgebung, von massiv bis grazil, zwischen einer Shore-Härte im Bereich von 30 bis 80 Shore A variieren.

Vorzugsweise wird die Shore-Härte dabei derart gewählt, dass die Steifigkeit des Vaginalpessars mit angeformter taschenartiger Vertiefung zwischen 30 bis 100 % der Steifigkeit eines Vaginalpessars ohne taschenartige Vertiefung und/oder eines Ringpessars mit etwa gleichem Umfang und/oder in etwa gleichem Durchmesser liegt.

Bevorzugt ist vorgesehen, dass jeweils wenigstens eine insbesondere gleich ausgebildete bzw. dimensionierte Vertiefung in jedem lateralen Seitenwandabschnitt vorgesehen ist, wobei, vorzugsweise, die Vertiefungen gegenüberliegend angeordnet sind. Dies ermöglicht ein verbessertes bzw. beidseitiges Anhaften des Vaginalpessars.

Es kann jedoch auch vorgesehen sein, dass zusätzlich oder alternativ wenigstens eine Vertiefung in dem posterioren Wandabschnitt vorgesehen bzw. ausgebildet ist. Nicht ausgeschlossen ist, dass zusätzlich oder alternativ wenigstens eine Vertiefung im anterioren Wandabschnitt ausgebildet ist.

Bevorzugt beträgt das Aufnahmevolumen der Vertiefung zwischen 1 und 10 Milliliter (ml), vorzugsweise zwischen 2 und 4 ml.

Alternativ oder zusätzlich beträgt die Öffnungsfläche der Vertiefung auf der Außenseite des Grundkörpers zwischen 1 und 8 cm², vorzugsweise zwischen 2 und 3 cm²,

Insbesondere kann, bezogen auf eine Seitenansicht des Vaginalpessars bzw. auf eine äußere Konturlinie des Vaginalpessars in der Seitenansicht, der Anteil der Öffnungsfläche der Vertiefung an der Gesamtkonturfläche des Vaginalpessars zwischen 20 % und 70 %, vorzugsweise zwischen 20 und 30 %, betragen.

Besonders bevorzugt ist die Vertiefung nach innen gewölbt bzw. kalottenartig ausgebildet und in Richtung zum Mittelpunkt der mittleren Öffnung des Grundkörpers des Pessars eingedrückt.

Die Konturlinie der Vertiefung ist mit Bezug auf eine Seitenansicht auf den die Vertiefung aufweisenden Wandabschnitt des Pessars vorzugsweise länglich, eiförmig, vorzugsweise elliptisch, ausgebildet. Es ist jedoch auch eine kreisförmige Ausbildung der inneren Kontur möglich.

Weitere Vorteile, Besonderheiten und/oder Merkmale ergeben sich aus der folgenden Zeichnungsbeschreibung. Es versteht sich, dass die oben beschriebenen und nachfolgend gezeigten Merkmale der unterschiedlichen Ausführungsformen der Erfindung bedarfsweise miteinander kombiniert werden können, auch wenn dies nicht im Einzelnen ausdrücklich erwähnt ist. Die Erfindung ist nicht auf die dargestellten Ausführungsbeispiele beschränkt. Die gewählte Absatzformatierung steht einer Kombination von Merkmalen aus unterschiedlichen Absätzen nicht entgegen.

Nachfolgend wird die Erfindung in Verbindung mit der Zeichnung anhand bevorzugter Ausführungsformen näher erläutert. Dabei zeigen die Figuren zunächst die unter den Nummern aufgeführten Eigenschaften, die dann noch näher erläutert werden.

Es zeigt:
- Fig. 1: eine perspektivische Ansicht eines erfindungsgemäßen Vaginalpessars gemäß einer ersten Ausführungsform,
- Fig. 2: eine schematische Draufsicht des Vaginalpessars gemäß Fig. 1,
- Fig. 3: eine Querschnittsansicht des Vaginalpessars aus Fig. 2 entlang der Schnittlinie III - III,
- Fig. 4: eine Vorderansicht des erfindungsgemäßen Vaginalpessars gemäß der ersten Ausführungsform,
- Fig. 5: eine Seitenansicht des erfindungsgemäßen Vaginalpessars gemäß der ersten Ausführungsform,
- Fig. 6: eine perspektivische Vorderansicht eines nicht erfindungsgemäßen Vaginalpessars,
- Fig. 7: eine perspektivische Seitenansicht des Vaginalpessars aus Fig. 6 ,
- Fig. 8: eine perspektivische Seitenansicht eines nicht erfindungsgemäßen Vaginalpessars,
- Fig. 9: eine Draufsicht des Vaginalpessars aus Fig. 8,
- Fig. 10: eine Draufsicht eines nicht erfindungsgemäßen Vaginalpessars,
- Fig. 11: eine perspektivische Seitenansicht des Vaginalpessars aus Fig. 10 ,
- Fig. 12: eine Draufsicht eines nicht erfindungsgemäßen Vaginalpessars,
- Fig. 13: eine perspektivische Hinteransicht des Vaginalpessars aus Fig. 12,
- Fig. 14: eine weitere Hinteransicht des Vaginalpessars aus Fig. 13,
- Fig. 15: eine perspektivische Ansicht eines nicht erfindungsgemäßen Vaginalpessars und
- Fig. 16: eine Seitenansicht des Vaginalpessars aus Fig. 15 .

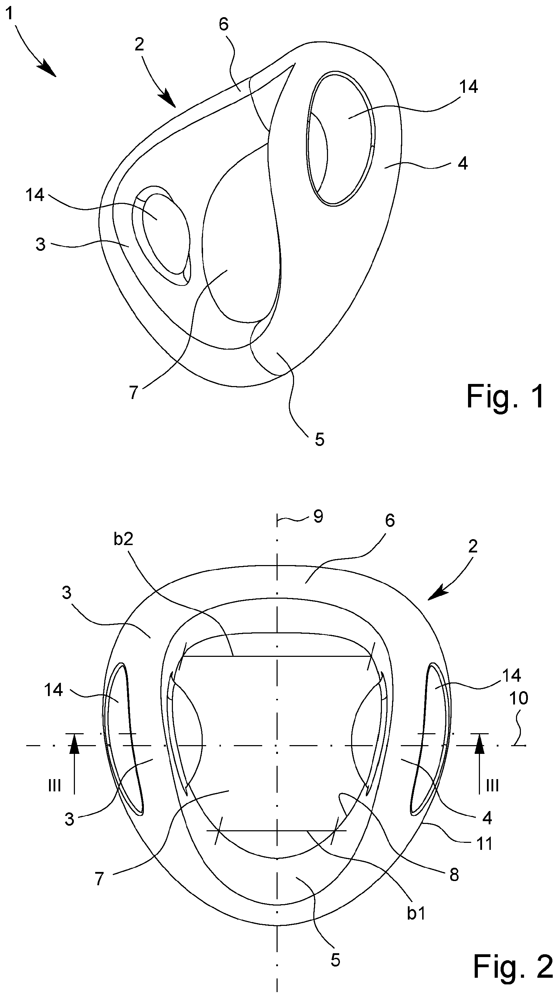

Die Figuren 1 bis 5 zeigen ein Vaginalpessar 1 gemäß einer ersten Ausführungsform, welches als vaginal einführbare Stütz-Struktur bei Senkungsbeschwerden, beispielsweise nach einer Geburt und/oder als Rückbildungs- bzw. Fixationshilfe, eingesetzt wird.

Das Vaginalpessar 1 weist einen umlaufend geschlossenen Grundkörper 2 auf, der zum vaginalen Einführen zu Zwecken der Abstützung im Introitus-Bereich (Eingang der Vagina) und/oder bei einer abgesenkten Gebärmutter ausgebildet bzw. dimensioniert ist.

Der Grundkörper 2 weist zwei laterale Seitenwandabschnitte 3, 4, einen vorderen bzw. anterioren Wandabschnitt 5 sowie einen hinteren, posterioren Wandabschnitt 6 auf.

In einem eingesetzten Zustand des Grundkörpers 2 befindet sich der anteriore Wandabschnitt 5 in einer zur Körpervorderseite gerichteten Orientierung bzw. Ausrichtung, wohingegen sich der hintere bzw. posteriore Wandabschnitt 6 in einer zur Körperhinterseite bzw. Rückenseite gerichteten Orientierung bzw. Ausrichtung befindet.

Die zwischen dem anterioren Wandabschnitt 5 und dem posterioren Wandabschnitt 6 befindlichen seitlichen bzw. lateralen Seitenwandabschnitte 3, 4 sind dementsprechend gegenüberliegenden Körperseiten, d.h. der linken bzw. rechten Körperseite, einer Patientin zugeordnet. Im eingesetzten Zustand liegt der Grundkörper 2 direkt bzw. außenseitig umlaufend an angrenzenden Gewebeabschnitten an, insbesondere derart, dass zu stützende Körperbereiche bzw. Organe, wie die Gebärmutter, entgegengesetzt zur Schwerkraftsrichtung mittels des Vaginalpessars 1 bzw. des Grundkörpers 2 gehalten bzw. abgestützt werden.

Der Grundkörper 2 weist eine mittlere Öffnung 7 auf, die in einer Querschnittsansicht quer zur Höhe des Grundkörpers 2 durch eine geschlossene, innere Konturlinie 8 der Wandabschnitte 3, 4, 5 und 6 begrenzt ist. Die Querschnittsansicht quer zur Höhe des Grundkörpers 2 ist letztlich parallel zu der in Fig. 2 dargestellten Draufsicht des Grundkörpers 2 zu verstehen.

Die innere Konturlinie 8 begrenzt eine im Wesentlichen eiförmig ovalförmige und von der Kreisform abweichende Öffnung 7, wie in Fig. 2 ersichtlich.

Die Abweichung von der Kreisform kann letztlich beliebig ausgebildet sein, wobei entlang der inneren Konturlinie 8 kreisförmige Kontur-Abschnitte vorgesehen sein können. Entlang der inneren Konturlinie 8 ist dann jedoch mindestens ein Abschnitt derart vorgesehen, dass sich, insbesondere in Bezug auf die in Fig. 2 gezeigte Draufsicht auf den Grundkörper 2, eine von der Kreisform abweichende Öffnung 7 einstellt. Die von der Kreisform abweichende Öffnung 7 kann asymmetrisch ausgebildet sein und letztlich als beliebiges Oval vorliegen, um eine von der Kreisform abweichende innere Konturlinie 8 auszubilden.

Beim dargestellten und bevorzugten Ausführungsbeispiel ist die innere Konturlinie 8 symmetrisch zu einer Mittellängsachse 9 des Grundkörpers 2 ausgebildet und weist, insbesondere, einen im Wesentlichen eiförmigen Verlauf auf.

Dargestellt und bevorzugt ist, dass die Konturlinie 8 ein spitzes bzw. kurzes Ende auf der Seite des anterioren Wandabschnitts 5 und ein langes bzw. stumpfes Ende auf der Seite des posterioren Wandabschnitts 6 aufweist. Dementsprechend weist die Öffnung 7 im Bereich des anterioren Wandabschnitts 5 eine geringere Breite, d.h. Öffnungsweite, als im Bereich des posterioren Wandabschnitts 6 auf.

Die unterschiedliche Breite der Öffnung 7 ist insbesondere in Bezug auf eine quer zur Mittellängsachse 9 verlaufende Mittelquerachse 10 der Öffnung 7 und/ oder des Grundkörpers 2 zu verstehen.

Mit Referenz auf denselben Abstand zur Mittelquerachse 10 weist die Öffnung 7 im Bereich des anterioren Wandabschnitts 5 eine verringerte Öffnungsbreite b1 als im Bereich des posterioren Wandabschnitts 6 mit einer größeren Breite b2 auf.

Ausgehend von der Mittelquerachse 10 nimmt die Weite bzw. Breite der Öffnung 7 hin zum anterioren Wandabschnitt 5 ab, wohingegen sich die Breite ausgehend von der Mittelquerachse 10 hin zum posterioren Wandabschnitt 6 zunächst erhöht und sich unmittelbar vor dem posterioren Wandabschnitt 6 dann stark reduziert.Der Grundkörper 2 weist eine der inneren Konturlinie 8 gegenüberliegende äußere Konturlinie 11 auf, wobei die äußere Konturlinie 11 zumindest im Wesentlichen und/oder abschnittsweise dem Verlauf der inneren Konturlinie 8 folgt. Mit anderen Worten ist der Grundkörper 2 zumindest im Wesentlichen ringartig mit aneinander angepassten, insbesondere zumindest abschnittsweise zueinander parallelen, Konturlinien 8, 11 ausgebildet.

Die innere Konturlinie 8 und/oder die äußere Konturlinie 11 ist bzw. sind zumindest im Wesentlichen spiegelsymmetrisch zur Mittellängsachse 9 und/oder zur Mittelquerachse 10 des Grundkörpers 2 ausgebildet. Bei einer Spiegelsymmetrie in Bezug auf die Mittellängsachse 9 ist dabei eine vorzugsweise eiförmige Öffnung 7 vorgesehen. Liegt die Spiegelsymmetrie zu beiden Mittelachsen 9, 10 vor, ist die Öffnung 7 bzw. sind die Konturlinien 8, 11 vorzugsweise ellipsenförmig ausgebildet. Die zu wählende Gestalt ist insofern vielfältig und kann im Hinblick auf den angestrebten Anwendungsfall individuell angepasst werden.

Die gegenüberliegenden Seitenwandabschnitte 3, 4 sind vorzugsweise im Wesentlichen gleichförmig konvex gebogen, insbesondere wobei die innere Konturlinie 8 und die äußere Konturlinie 11 im Bereich der lateralen Seitenwandabschnitte 3, 4 jeweils gleichförmig konvex gebogen sind.

Es ist jedoch auch eine geradlinige Ausbildung der inneren Konturlinie 8 und/oder der äußeren Konturlinie 11 im Bereich der Seitenwandabschnitte 3, 4 möglich.

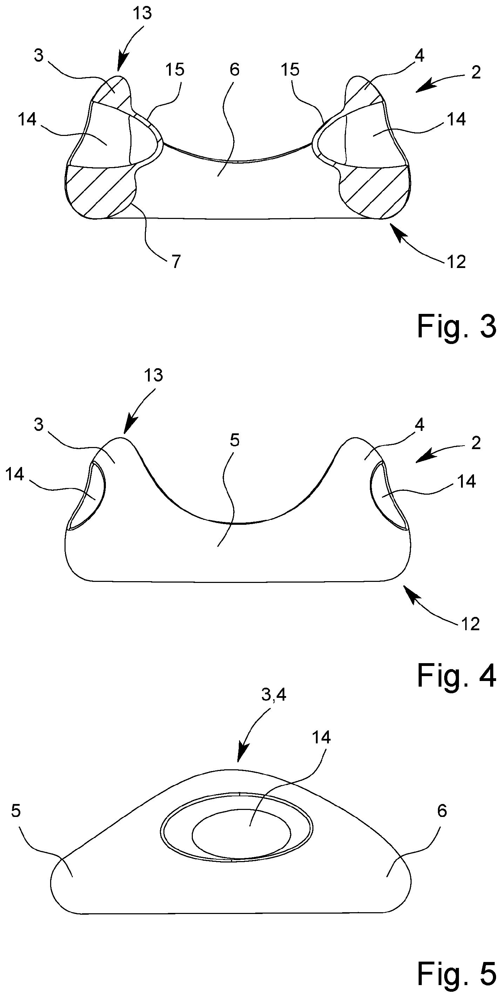

Wie anhand von Fig. 3 ersichtlich, nimmt die Dicke der Wandabschnitte, vorzugsweise der Seitenwandabschnitte 3, 4, ausgehend von einer proximalen Außenseite 12 des Grundkörpers 2 in Richtung zu einer distalen Außenseite 13 des Grundkörpers 2 hin ab. Die proximale Außenseite 12 ist im eingesetzten Zustand zur Körpermitte hin gerichtet, wohingegen die distale Außenseite 13 von der Körpermitte weg gerichtet ist. Die zur distalen Außenseite 13 hin abnehmende Dicke des Grundkörpers 2 gestattet eine weiterführende Verbesserung bzw. Anpassung des Grundkörpers 2 im Einsatzbereich, insbesondere im Hinblick an eine dicht anliegende Kontaktierung des Grundkörpers 2 mit dem angrenzenden Gewebe. Dadurch wird eine sichere Abstützung der Gewebeabschnitte an das Vaginalpessar 1 gewährleistet. Wie anhand von Fig. 1 verdeutlicht, weist der Grundkörper 2 eine sich in Umfangsrichtung vorzugsweise stetig ändernde Höhe auf. Bei einer stetig ändernden Höhe ist vorzugsweise kein sprunghafter Übergang bzw. keine sprunghafte Änderung der Höhe in Umfangsrichtung des Grundkörpers 2 vorgesehen.

Die Seitenwandabschnitte 3, 4 sind gegenüber dem anterioren Wandabschnitt 5 und dem posterioren Wandabschnitt 6 erhöht.

Wie in den Figuren 4 und 5 verdeutlicht, weisen die Seitenwandabschnitte 3, 4 dieselbe maximale Höhe auf. Insbesondere bilden die lateralen Seitenwandabschnitte 3, 4 gegenüber dem posterioren Wandabschnitt 5 und dem anterioren Wandabschnitt 6 hervorstehende Laschen mit bogenförmiger Oberseite aus. Dies erleichtert das Einführen des Grundkörpers 2 in die Vagina. Die lateralen Seitenwandabschnitte 3, 4 können jedoch auch unterschiedliche Höhen aufweisen.

Der posteriore Wandabschnitt 5 und der anteriore Wandabschnitt 6 weisen vorzugsweise dieselbe, gegenüber den Seitenwandabschnitten 3, 4 verringerte, insbesondere minimale, Höhe auf. Jedoch ist auch eine abweichende Höhe zwischen dem anterioren Wandabschnitt 5 und dem posterioren Wandabschnitt 6 möglich.

Der Grundkörper 2 weist eine auf der Außenseite des Grundkörpers 2 geöffnete, taschenförmige Vertiefung 14 auf, um eine bessere Anheftung für anliegendes Gewebe im Einsetzzustand des Vaginalpessars 1 zu bilden. Die Seitenwände 3, 4 weisen jeweils eine Vertiefung 14 auf, die radial nach innen und/oder radial in die Öffnung 7 hineinragend ausgebildet ist, wie in Fig. 2 dargestellt.

Der Aspekt der taschenförmigen Vertiefung 14 wird im Zusammenhang mit einer eiförmigen inneren Konturlinie 8 realisiert. Auf diese Weise lässt sich in Kombination eine verbesserte Anpassbarkeit bei gleichzeitig sicherer Anbindung des Vaginalpessars 1 erzielen.

Fig. 3 verdeutlicht, dass die Vertiefungen 14 auf der Innenseite des Grundkörpers 2, nämlich auf der Seite der mittleren Öffnung 7, geschlossen sind. Dabei sind die Vertiefungen 14 auf der Innenseite des Grundkörpers 2 durch einen weiteren bzw. dünnwandigen Wandabschnitt 15 begrenzt, der gegenüber den an die Vertiefungen 14 angrenzenden Wandabschnitten, vorzugsweise Seitenwandabschnitten 3,4 des Grundkörpers 2 eine verringerte Materialstärke und/oder in der Elastizität aufweist. Besonders bevorzugt sind die Vertiefungen 14 an den Wandabschnitten 15 nach innen ausgestülpt.

Vorzugsweise weist der dünnwandige Wandabschnitt 15 gegenüber dem an die Vertiefung 14 angrenzenden Wandabschnitt, vorzugsweise einem zugeordneten Seitenwandabschnitt 3, 4, eine um wenigstens 40 %, vorzugsweise wenigstens 60 %, besonders bevorzugt wenigstens 80 %, ganz besonders bevorzugt wenigstens 90 %, verringerte Materialstärke auf.

Durch die dünnwandigen Wandabschnitte 15 lässt sich im Vorfeld des Einsatzzustandes eine Verringerung des Saugvolumens innerhalb der Vertiefungen 14 ausbilden, um auf dieser Grundlage eine Saugwirkung zu erzielen, wenn sich die Wandabschnitte 15 im Einsatzzustand wieder radial nach innen aufgrund ihrer Elastizität ausrichten und sich dadurch ein Unterdruck innerhalb der Vertiefungen 14 aufbaut.

Die dünnwandigen Wandabschnitte 15 weisen, insbesondere im Zentrum, vorzugsweise eine Dicke im Bereich von 0,1 mm bis 4 mm, vorzugsweise von 0,5 mm bis 3 mm, insbesondere von 0,5 mm bis 2 mm, besonders bevorzugt von 0,5 mm bis 1 mm, auf.

Die Herstellung des Grundkörpers 2 kann durch herkömmliche Produktionsverfahren erfolgen, beispielsweise durch Spritzguss, Gießen und/oder Pressen mit einem elastomeren Kunststoff- und/oder Silikon

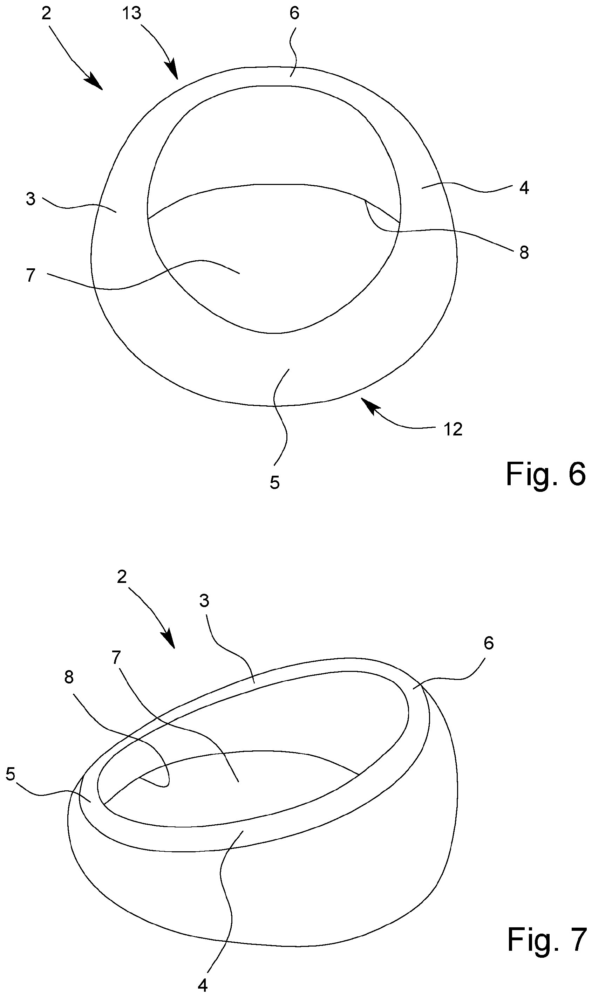

Bei der in Fig. 6 gezeigten Ausführungsform weist der Grundkörper 2 eine sich in Umfangsrichtung stetig ändernde Höhe auf, wobei eine minimale Höhe am anterioren Wandabschnitt 5 und eine maximale Höhe am posterioren Wandabschnitt 6 erreicht wird. Die sich stetig ändernde Höhe ist vorzugsweise in Bezug auf die distale Außenseite 13 des Grundkörpers 2 zu verstehen, wobei die proximale Außenseite 12 vorzugsweise eine gleichbleibende Höhe aufweist und/oder kleine Höhenveränderungen in Umfangsrichtung vorsieht.

Die distale Außenseite 13 bzw. Oberseite des Grundkörpers 2 ist vorzugsweise abgeschrägt ausgebildet.

Es versteht sich, dass die zuvor im Hinblick auf die erste Ausführungsform gezeigten Merkmale, sofern diese nicht in Widerspruch zur zweiten Ausführungsform gemäß Fig. 6 das Vaginalpessars 1 stehen, gleichermaßen auch für das Vaginalpessar 1 gemäß der zweiten Ausführungsform entsprechend gelten.

Dementsprechend weist, mit Bezug auf Fig. 7, das Vaginalpessar 1 gemäß der zweiten Ausführungsform eine mittlere Öffnung 7 auf, deren innere Konturlinie 8 der Wandabschnitte 3, 4, 5 und 6 eine im Wesentlichen ovalförmige und von der Kreisform abweichende Gestalt aufweist.

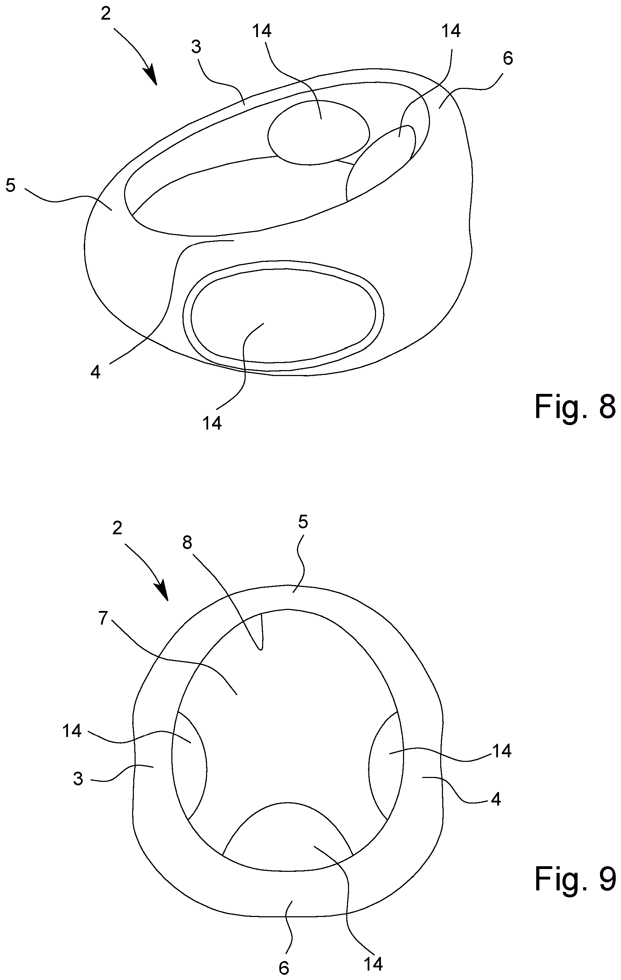

Die in den Fig. 8 und 9 gezeigte dritte Ausführungsform eines Vaginalpessars 1 entspricht im Wesentlichen der zweiten Ausführungsform, wobei der Grundkörper 2 mehr als zwei, beim Darstellungsbeispiel drei, Vertiefungen 14 aufweist. Dabei befindet sich je eine Vertiefung 14 an den gegenüberliegenden Seitenwandabschnitten 3, 4. Zusätzlich ist auch eine weitere Vertiefung 14 am posterioren Wandabschnitt 6 angeordnet.

Es versteht sich jedoch, dass, wie im Zusammenhang mit der zweiten Ausführungsform beschrieben, lediglich zwei Vertiefungen 14 an den gegenüberliegenden Seitenwandabschnitten 3, 4 vorgesehen sein können.

Wie in Fig. 9 ersichtlich, können die Vertiefungen 14 unterschiedlich groß ausgebildet sein und/oder unterschiedlich große Aufnahmevolumina aufweisen. Besonders bevorzugt sind die den Seitenwänden 3, 4 zugeordneten Vertiefungen 14 gleichdimensioniert, wohingegen die dem posterioren Wandabschnitt 6 zugeordnete Vertiefung 14 ein demgegenüber größeres Aufnahmevolumen aufweist.

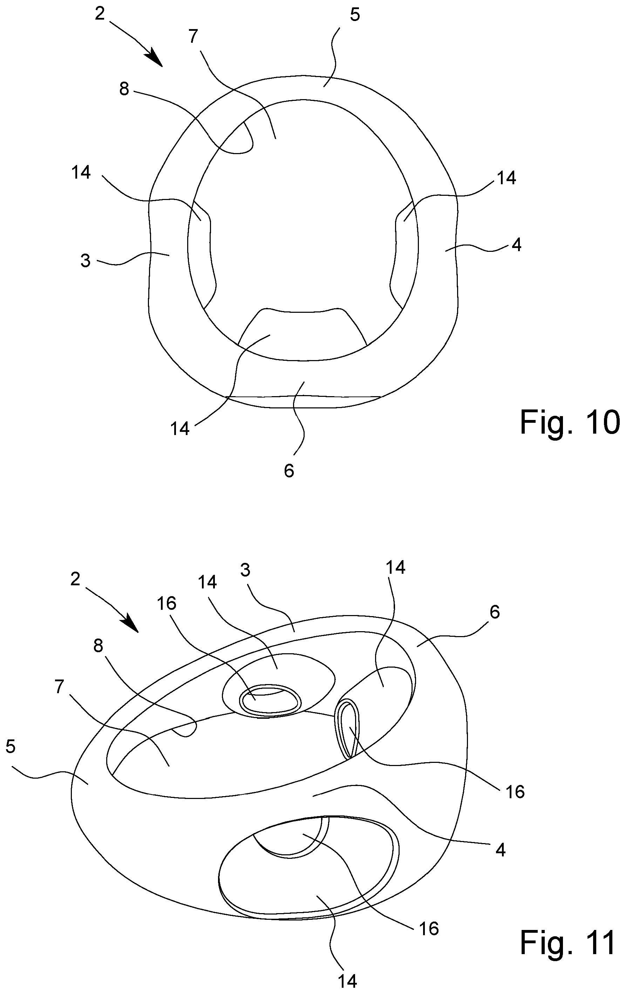

Bei der in den Fig. 10 und 11 gezeigten nicht erfindungsgemäßen Ausführungsform ist vorgesehen, dass wenigstens eine Vertiefung 14 radial bzw. innenseitig geöffnet ist. Dementsprechend weist wenigstens eine, vorzugsweise jede, Vertiefung 14 eine Durchbrechung 16 auf, wie in Fig. 11 illustriert.

Trotz der Durchbrechungen 16 ist eine ausreichende Ansaugwirkung im eingesetzten Zustand des Vaginalpessars 1 sichergestellt. Gleichzeitig ergibt sich eine weiterführend erhöhte Elastizität, da der dünnwandige Wandabschnitt 15 der Vertiefung 14 durch die Durchbrechung 16 weiterführend geschwächt und/oder elastischer ausgebildet ist. Gleichzeitig ermöglicht die Durchbrechung 16 einen Flüssigkeitsablauf aus dem Grundkörper 2.

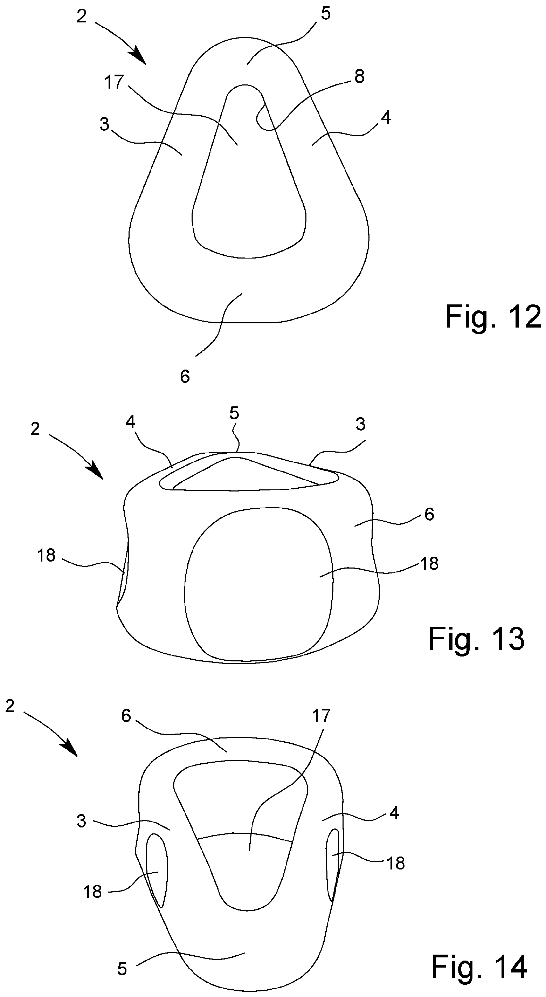

Nachfolgend werden anhand der Fign. 12 bis 14 nicht erfindungsgemäße Ausführungsformen beschrieben, bei denen die innere Konturlinie 8 durch eine im Wesentlichen trapezoide Öffnung 17 begrenzt ist.

Die Konturlinie 8 ist im Bereich der gegenüberliegenden Seitenwandabschnitte 3, 4 im Wesentlichen geradlinig ausgebildet. Es ist jedoch auch eine vorzugsweise geringfügig ausgebildete konvexe Ausbildung der Konturlinie 8 im Bereich der gegenüberliegenden Seitenwandabschnitte 3, 4 möglich, wobei weiterhin eine trapezoide Öffnung 17 vorliegt. Die Konturlinie 8 ist im Bereich des anterioren Wandabschnitts 5 sowie des posterioren Wandabschnitts 6 entsprechend konvex gekrümmt. Es ist jedoch auch eine geradlinige Ausbildung des anterioren und/oder des posterioren Wandabschnitts 5, 6 möglich, insbesondere um eine exakt trapezoide innere Konturlinie 8 bzw. mittlere Öffnung 17 auszubilden. Bei der in Fig. 13 gezeigten sechsten Ausführungsform weist der Grundkörper 2 mit der trapezoiden inneren Öffnung 17 bzw. inneren Konturlinie 8 wenigstens eine Einkerbung 18 auf. Beim dargestellten und bevorzugten Ausführungsbeispiel ist diese Einkerbung 18 am posterioren Wandabschnitt 6 ausgebildet. Zusätzlich ist bei dieser Ausführungsform, wie in Fig. 14 dargestellt, auch eine weitere Einkerbung 18 jeweils an den Seitenwandabschnitten 3, 4 vorgesehen.

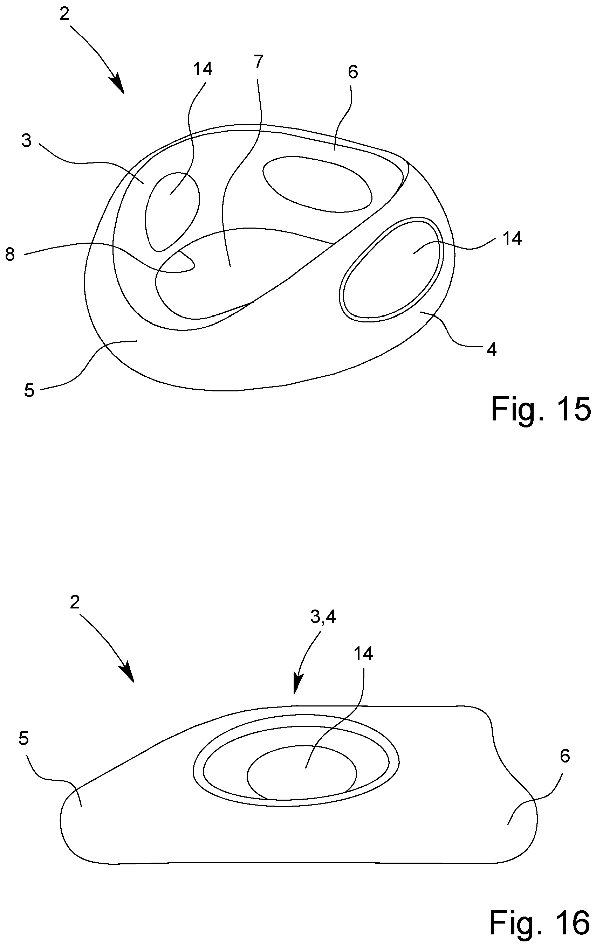

Bei der in den Figuren 15 und 16 dargestellten siebten Ausführungsform weisen der posteriore Wandabschnitt 6 sowie die Seitenwandabschnitte 3, 4 dieselbe, maximale Höhe auf. Insbesondere bildet der posteriore Wandabschnitt 6 zusammen mit den Seitenwandabschnitten 3, 4 einen Plateaubereich aus.

Am anterioren Wandabschnitt 5 liegt die minimale Höhe des Grundkörpers 2 vor, insbesondere wobei ausgehend vom anterioren Wandabschnitt 5 die Höhe des Grundkörpers 2 beidseitig in Richtung zu den Seitenwandabschnitten 3, 4 ansteigt und schließlich an den Seitenwandabschnitten 3, 4 die maximale Höhe erreicht.

Beim dargestellten Ausführungsbeispiel weisen die Seitenwandabschnitte 3, 4 sowie der posteriore Wandabschnitt 6 jeweils eine geschlossene Vertiefung 14 auf.

Nachfolgende Merkmale können vorzugsweise in Bezug auf sämtliche Ausführungsformen gleichermaßen realisiert werden:
Der Grundkörper 2 weist eine Breite im Bereich von 50 bis 80 mm, vorzugsweise von 55 bis 65 mm, ganz besonders bevorzugt von etwa 60 mm, auf.

Der Grundkörper 2 weist eine Länge im Bereich von 55 bis 90 mm, vorzugsweise im Bereich von 60 bis 75 mm auf. Der Grundkörper 2 weist eine maximale Höhe im Bereich von 15 bis 45 mm, vorzugsweise im Bereich von 25 bis 35 mm auf.

Der Grundkörper 2 weist eine minimale Höhe im Bereich von 5 bis 15 mm, vorzugsweise im Bereich von 8 bis 12 mm auf.

Die maximale Breite, d.h. Öffnungsweite, der Öffnung 7 und/oder 17 liegt im Bereich von 30 bis 50 mm, vorzugsweise im Bereich von 35 bis 40 mm.

Der Grundkörper 2 weist eine vordere, vorzugsweise wulstartige, Verstärkung auf, insbesondere wobei die Verstärkung am anterioren Wandabschnitt 5 ausgebildet ist.

Die Vertiefung 14 bzw. das Aufnahmevolumen der Vertiefung 14 ist halbellipsenförmig und/oder kalottenartig ausgebildet. Das Aufnahmevolumen kann auch halbkugelförmig und/oder halbelliptisch ausgebildet sein.Einzelne Aspekte und Merkmale der vorliegenden Erfindung, insbesondere der verschiedenen Ausführungsformen, können einzeln und in verschiedenen Kombinationen realisierbar und vorteilhaft sein.

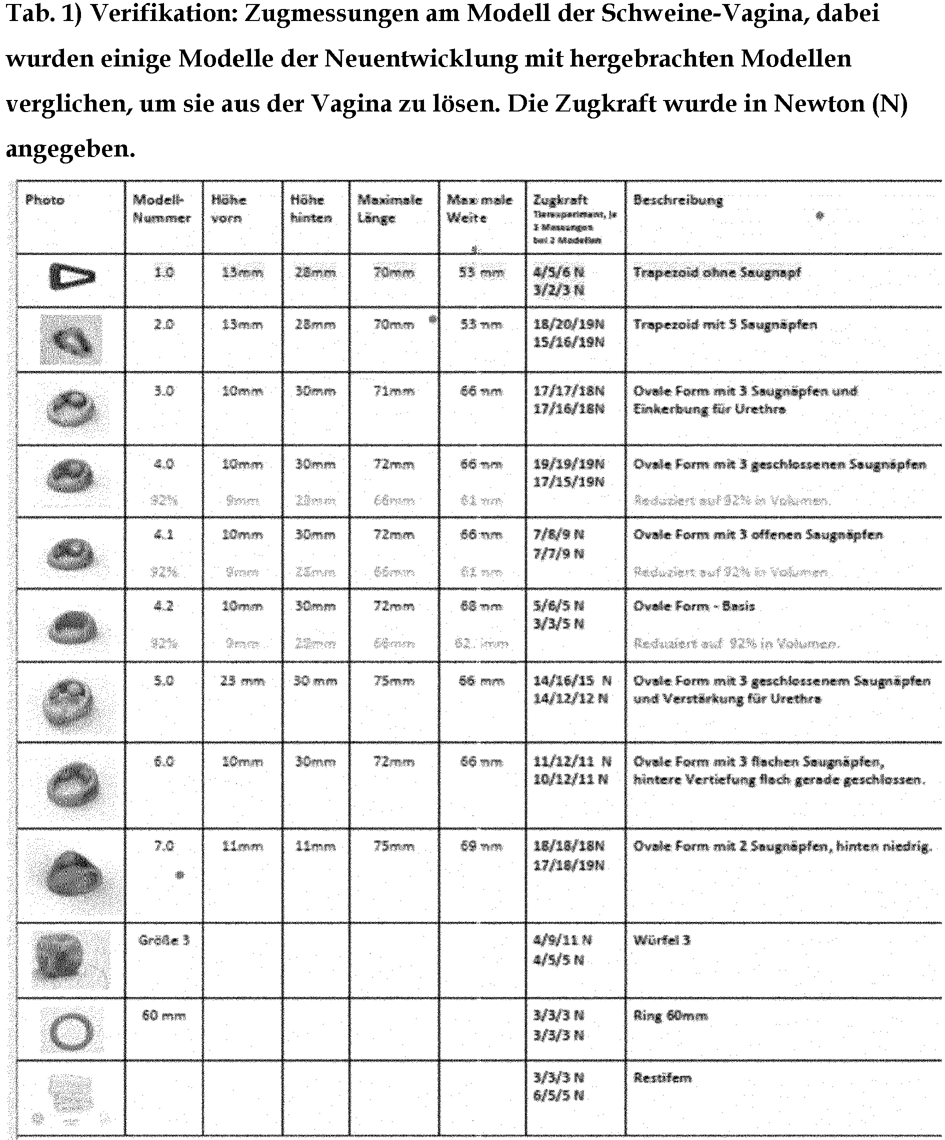

### Validierung: Herstellung von Sonderanfertigungen für einzelne Patientinnen nach Anfrage von Ärzten unter Vertraulichkeitsbestimmungen auch im Rahmen eines ZIM Projektes und Zusammenfassung der Erkenntnisse:

### Genereller Eindruck:

1) Die Applikation von Modellen mit Einbuchtungen an den Seitenwänden ist besonders für jüngere Frauen mit Beschwerden nach Geburten geeignet, da die Modelle eine hohe Haftkraft haben und zusätzlich auch das Ablaufen des Fluors ermöglichen.
2) Die Applikation mit zusätzlicher Einbuchtung an der Hinterwand ist eher für ältere Patientinnen nach Senkungsoperationen zur zusätzlichen Stütze geeignet,
3) Die Senkungsbeschwerden wurden in beiden Gruppen deutlich verbessert
4) Alle eingesetzten Modelle waren zum "Self-Management" geeignet, d.h., sie konnten einfach durch die Patientin selbst gewechselt werden.
5) Während einer Spezialisten-Untersuchung (Prof. Dietz) zeigten sich auch im transperinealen Ultraschall Kontraktionen der Muskulatur des Beckenbodens bei Risikopatientinnen mit Vorschäden und eine Reduktion der Hiatus-Öffnung.

### Bezugszeichenliste:

- 1: Vaginalpessar
- 2: Grundkörper
- 3: Seitenwandabschnitt
- 4: Seitenwandabschnitt
- 5: anteriore Wandabschnitt
- 6: posteriore Wandabschnitt
- 7: mittlere Öffnung
- 8: innere Konturlinie
- 9: Mittellängsachse
- 10: Mittelquerachse
- 11: äußere Konturlinie
- 12: proximale Außenseite
- 13: distale Außenseite
- 14: Vertiefung
- 15: dünnwandige Wandabschnitt
- 16: Durchbrechung
- 17: trapezoide Öffnung
- 18: Einkerbung
- b1: Breite
- b2: Breite

### Literatur

1) Harvey MA, Lemieux MC, Robert M, Schulz JA. Guideline No. 411: Vaginal Pessary Use. J Obstet Gynaecol Can. 2021;43(2):255-66 e1. doi: 10.1016/j.jogc.2020.11.013 .
2) Tunn R, Albrich S, Beilecke K, Kociszewski J, Lindig-Knopke C, Reisenauer C, et al. Interdisciplinary S2k Guideline: Sonography in Urogynecology: Short Version - AWMF Registry Number: 015/055. Geburtshilfe Frauenheilkd. 2014;74(12):1093-8. doi: 10.1055/s-0034-1383044 .
3) Sante HAd. Prolapsus genital de la femme - des solutions pour le traiter. 2022. doi: https://www.has-sante.fr/upload/docs/application/pdf/2022-04/reco443_fiche_patient_principale_prolapsus_cd_2022_04_28_v0.pdf. Accessed:21.12.2022.
4) National Institute for Health and Care Excellence. NICE Guidance - Urinary incontinence and pelvic organ prolapse in women: management: (c) NICE (2019) Urinary incontinence and pelvic organ prolapse in women: management. BJU Int. 2019;123(5):777-803. doi: 10.1111/bju.14763 .
5) Lone F, Thakar R, Sultan AH. One-year prospective comparison of vaginal pessaries and surgery for pelvic organ prolapse using the validated ICIQ-VS and ICIQ-UI (SF) questionnaires. Int Urogynecol J. 2015;26(9):1305-12. doi: 10.1007/ s00192-015-2686-9 .
6) Dietz HP, Walsh C, Subramaniam N, Friedman T. Levator avulsion and vaginal parity: do subsequent vaginal births matter? Int Urogynecol J. 2020;31(11):2311-5. doi: 10.1007/s00192-020-04330-4 .
7) Hilde G, Steer-Jensen J, · Siafarikas F, Ellström M, Bø K: Postpartum pelvic floor muscle training, levator ani avulsion and levator hiatus area: a randomized trial. Int Urogynecol J 2023; 34:413-23. Doi: 10.1007/s00192-022-05406-z

## Patentansprüche

1. Vaginalpessar (1), mit einem umlaufend geschlossenen Grundkörper (2), wobei der Grundkörper (2) zwei laterale Seitenwandabschnitte (3, 4), einen vorderen, anterioren Wandabschnitt (5) und einen hinteren, posterioren Wandabschnitt (6) aufweist und wobei eine mittlere Öffnung (7) des Grundkörpers (2) in einer Querschnittsansicht quer zur Höhe des Grundkörpers (2) durch eine geschlossene, innere Konturlinie (8) der Wandabschnitte (3, 4, 5, 6) begrenzt wird, wobei die innere Konturlinie (8) der Wandabschnitte (3, 4, 5, 6) eine im Wesentlichen eiförmige und von der Kreisform abweichende Öffnung (7) begrenzt, wobei die Öffnung (7) abgerundete Kanten mit einem spitzen Ende auf dem vorderen Wandabschnitt (5) und einem stumpfen Ende auf dem hinteren Wandabschnitt (6) aufweist, **dadurch gekennzeichnet, dass** der Grundkörper (2) zwei laterale, laschenförmige Seitenwandabschnitte (3, 4) aufweist, die gegenüber dem anterioren Wandabschnitt (5) und dem posterioren Wandabschnitt (6) erhöht sind, wobei die Seitenwandabschnitte (3, 4) jeweils eine auf der Außenseite des Grundkörpers (2) geöffnete, taschenartige, radial nach innen und/oder in die Öffnung (7) hineinragende Vertiefung (14) aufweisen, um eine Saug- bzw. Haftzone für anliegendes Gewebe im Einsetzzustand des Vaginalpessars (1) zu bilden.

2. Vaginalpessar (1) nach Anspruch 1, **dadurch gekennzeichnet, dass** der vordere, anteriore Wandschnitt (5) und/oder der hintere, posteriore Wandabschnitt (6) auf der Seite der Öffnung (7) in der Querschnittsansicht im Wesentlichen konvex gebogen ist, wobei der vordere, anteriore Wandschnitt (5) und/oder der hintere, posteriore Wandabschnitt (6) auf der Seite der Öffnung (7) in der Querschnittsansicht im Wesentlichen unterschiedlich konvex gebogen ist.

3. Vaginalpessar (1) nach Anspruch 1 oder 2, **dadurch gekennzeichnet, dass** die innere Konturlinie (8) der Wandabschnitte (3, 4, 5, 6) spiegelsymmetrisch zur Mittellängsachse (9) und/oder zur Mittelquerachse (10) ist und/oder dass die lateralen Seitenwandabschnitte (3, 4) auf der Seite der Öffnung (7) im Wesentlichen konvex gebogen sind.

4. Vaginalpessar (1) nach einem der vorangehenden Ansprüche, **dadurch gekennzeichnet, dass** der Grundkörper (2) durch umlaufend ineinander übergehende Wandabschnitte (3, 4, 5, 6) gebildet wird und/oder dass die Konturlinie (8) entlang der Öffnung (7) einen durchgehend konvexen Verlauf aufweist, wobei die Konturlinie (8) im Bereich des vorderen (anterioren) Wandschnitts (5) stärker gekrümmt und/oder konvex gebogen ist als im Bereich des hinteren (posterioren) Wandabschnitts (6) und/oder dass die Dicke der Wandabschnitte (3, 4, 5, 6), vorzugsweise der Seitenwandabschnitte (3, 4), ausgehend von einer proximalen Außenseite (12) des Grundkörpers (2) in Richtung zu einer distalen Außenseite (13) des Grundkörpers (2) abnimmt.

5. Vaginalpessar (1) nach einem der vorhergehenden Ansprüche, **dadurch gekennzeichnet, dass** die Vertiefung (14) auf einer Innenseite eines Seitenwandabschnitts (3, 4) auf Seite der mittleren Öffnung (7) ausgebildet ist.

6. Vaginalpessar (1) nach einem der vorangehenden Ansprüche, **dadurch gekennzeichnet, dass** die Vertiefung (14) auf einer Innenseite des Grundkörpers (2) geschlossen oder geöffnet ist, insbesondere auf Seite der mittleren Öffnung (7), und/oder dass die Vertiefung (14) radial nach innen gerichtet ist, und/oder dass die Vertiefung (14) auf einer Innenseite des Grundkörpers (2) durch einen dünnwandigen Wandabschnitt (15) begrenzt wird, der gegenüber den an die Vertiefung (14) angrenzenden Wandabschnitten, insbesondere Seitenwandabschnitten (3, 4), des Grundkörpers (2) nach innen ausgestülpt ist.

7. Vaginalpessar (1) nach einem der vorangehenden Ansprüche, **dadurch gekennzeichnet, dass** die Vertiefung (14) auf einer Innenseite des Grundkörpers (2) durch einen dünnwandigen Wandabschnitt (15) begrenzt wird, der gegenüber an die Vertiefung (14) angrenzende Wandabschnitt, insbesondere Seitenwandabschnitten (3, 4), des Grundkörpers (2) eine höhere Elastizität und/oder eine geringere Wanddicke aufweist, und/oder dass jeweils wenigstens eine Vertiefung (14) in jedem lateralen Seitenwandabschnitt (3, 4) vorgesehen ist, wobei, vorzugsweise, die Vertiefungen (14) gegenüberliegend angeordnet sind.

8. Vaginalpessar (1) nach einem der vorangehenden Ansprüche, **dadurch gekennzeichnet, dass** das Aufnahmevolumen der Vertiefung (14) zwischen 1 und 10 Milliliter, vorzugsweise zwischen 2 und 4 Milliliter, beträgt und/oder dass eine Öffnungsfläche der Vertiefung (14) zwischen 1 und 8 cm 2, vorzugsweise zwischen 2 und 3 cm 2 , beträgt und/oder dass eine Öffnungsfläche der Vertiefung (14) in einer Seitenansicht eine konvexe und/oder von der Kreisform abweichende ovale Innenkontur aufweist.

## Claims

1. Vaginal pessary (1) with a circumferentially closed base body (2), wherein the base body (2) has two lateral side wall sections (3, 4), a front, anterior wall section (5) and a rear, posterior wall section (6), and wherein a central opening (7) of the base body (2) is bounded in a cross-sectional view transverse to the height of the base body (2) by a closed, inner contour line (8) of the wall sections (3, 4, 5, 6), wherein the inner contour line (8) of the wall sections (3, 4, 5, 6) delimits an essentially egg-shaped opening (7) that deviates from a circular shape, wherein the opening (7) has rounded edges with a pointed end on the front wall section (5) and a blunt end on the rear wall section (6), **characterized in that** the base body (2) has two lateral, flap-shaped side wall sections (3, 4) which are raised relative to the anterior wall section (5) and the posterior wall section (6), wherein the side wall sections (3, 4) each have a pocket-like recess (14) which is open on the outside of the base body (2) and projects radially inwards and/or into the opening (7) in order to form a suction or adhesion zone for adjacent tissue when the vaginal pessary (1) is inserted.

2. Vaginal pessary (1) according to claim 1, **characterized in that** the front, anterior wall section (5) and/or the rear, posterior wall section (6) on the side of the opening (7) is essentially convexly curved in cross-sectional view, wherein the front, anterior wall section (5) and/or the rear wall section (6) on the side of the opening (7) being substantially differently curved in cross-section.

3. Vaginal pessary (1) according to claim 1 or 2, **characterized in that** the inner contour line (8) of the wall sections (3, 4, 5, 6) is mirror-symmetrical to the central longitudinal axis (9) and/or to the central transverse axis (10) and/or that the lateral side wall sections (3, 4) on the side of the opening (7) are essentially convexly curved.

4. Vaginal pessary (1) according to one of the preceding claims, **characterized in that** the base body (2) is formed by circumferentially merging wall sections (3, 4, 5, 6) and/or that the contour line (8) along the opening (7) has a continuously convex course, wherein the contour line (8) is more strongly curved and/or convexly bent in the region of the front (anterior) wall section (5) than in the region of the rear (posterior) wall section (6) and/or that the thickness of the wall sections (3, 4, 5, 6), preferably the side wall sections (3, 4), decreases from a proximal outer side (12) of the base body (2) towards a distal outer side (13) of the base body (2).

5. Vaginal pessary (1) according to one of the preceding claims, **characterized in that** the recess (14) is formed on an inner side of a side wall section (3, 4) on the side of the central opening (7).

6. Vaginal pessary (1) according to one of the preceding claims, **characterized in that** the recess (14) on an inner side of the base body (2) is closed or open, in particular on the side of the central opening (7), and/or that the recess (14) is directed radially inward, and/or that the recess (14) on an inner side of the base body (2) is bounded by a thin-walled wall section (15) which is everted inwards relative to the wall sections, in particular side wall sections (3, 4), of the base body (2) adjacent to the recess (14).

7. Vaginal pessary (1) according to one of the preceding claims, **characterized in that** the recess (14) on an inner side of the base body (2) is bounded by a thin-walled wall section (15), the wall section adjacent to the recess (14), in particular side wall sections (3, 4) of the base body (2) have a higher elasticity and/or a lower wall thickness, and/or that at least one recess (14) is provided in each lateral side wall section (3, 4), wherein, preferably, the recesses (14) are arranged opposite each other.

8. Vaginal pessary (1) according to one of the preceding claims, **characterized in that** the capacity of the recess (14) is between 1 and 10 milliliters, preferably between 2 and 4 milliliters, and/or that an opening area of the recess (14) is between 1 and 8 cm², preferably between 2 and 3 cm², and/or that an opening area of the recess (14) has a convex and/or oval inner contour deviating from the circular shape in a side view.

## Revendications

1. Pessaire vaginal (1) comprenant un corps de base (2) fermé sur son pourtour, le corps de base (2) présentant deux parties latérales (3, 4), une partie de paroi avant (5) et une partie de paroi arrière (6), et une ouverture centrale (7) du corps de base (2) étant délimitée, dans une vue en coupe transversale perpendiculaire à la hauteur du corps de base (2), par une ligne de contour intérieure fermée (8) des parties de paroi (3, 4, 5, 6), la ligne de contour intérieure (8) des sections de paroi (3, 4, 5, 6) délimitant une ouverture (7) essentiellement ovoïde et s'écartant de la forme circulaire, l'ouverture (7) présentant des bords arrondis avec une extrémité pointue sur la section de paroi avant (5) et une extrémité émoussée sur la partie de paroi arrière (6), **caractérisé en ce que** le corps de base (2) présente deux parties de paroi latérales en forme de languettes (3, 4) qui sont surélevées par rapport à la partie de paroi antérieure (5) et à la partie de paroi postérieure (6), les parties de paroi latérales (3, 4) présentant chacune un renfoncement (14) en forme de poche, ouvert sur la face extérieure du corps de base (2), s'étendant radialement vers l'intérieur et/ou dans l'ouverture (7), afin de former une zone d'aspiration ou d'adhérence pour les tissus adjacents lorsque le pessaire vaginal (1) est en place.

2. Pessaire vaginal (1) selon la revendication 1, **caractérisé en ce que** la partie avant (5) de la paroi et/ou la partie arrière (6) de la paroi, du côté de l'ouverture (7), sont sensiblement courbées de manière convexe en vue en coupe transversale, la partie avant (5) de la paroi (5) et/ou la partie de paroi postérieure (6) du côté de l'ouverture (7) présentant une courbure sensiblement différente dans la vue en coupe transversale.

3. Pessaire vaginal (1) selon la revendication 1 ou 2, **caractérisé en ce que** la ligne de contour interne (8) des sections de paroi (3, 4, 5, 6) est symétrique par rapport à l'axe longitudinal central (9) et/ou à l'axe transversal central (10) et/ou **en ce que** les parties latérales de la paroi (3, 4) sont courbées de manière essentiellement convexe du côté de l'ouverture (7).

4. Pessaire vaginal (1) selon l'une des revendications précédentes, **caractérisé en ce que** le corps de base (2) est formé par des sections de paroi (3, 4, 5, 6) et/ou **en ce que** la ligne de contour (8) le long de l'ouverture (7) présente un tracé convexe continu, la ligne de contour (8) étant plus courbée et/ou convexe dans la zone de la section de paroi avant (antérieure) (5) que dans la zone de la section de paroi arrière (postérieure) (6) et/ou **en ce que** l'épaisseur des sections de paroi (3, 4, 5, 6), de préférence des sections de paroi latérales (3, 4), diminue à partir d'un côté extérieur proximal (12) du corps de base (2) en direction d'un côté extérieur distal (13) du corps de base (2).

5. Pessaire vaginal (1) selon l'une des revendications précédentes, **caractérisé en ce que** le renfoncement (14) est formé sur une face intérieure d'une partie de paroi latérale (3, 4) du côté de l'ouverture centrale (7).

6. Pessaire vaginal (1) selon l'une des revendications précédentes, **caractérisé en ce que** le renfoncement (14) est fermé ou ouvert sur une face intérieure du corps de base (2), en particulier du côté de l'ouverture centrale (7), et/ou **en ce que** le renfoncement (14) est orienté radialement vers l'intérieur, et/ou que le renfoncement (14) est délimité sur une face intérieure du corps de base (2) par une partie de paroi à paroi mince (15) qui est retournée vers l'intérieur par rapport aux parties de paroi adjacentes au renfoncement (14), en particulier aux parties de paroi latérales (3, 4), du corps de base (2).

7. Pessaire vaginal (1) selon l'une des revendications précédentes, **caractérisé en ce que** le renfoncement (14) sur une face intérieure du corps de base (2) est délimité par une partie de paroi à paroi mince (15), la partie de paroi adjacente au renfoncement (14), en particulier les parties de paroi latérales (3, 4) du corps de base (2) présentent une élasticité plus élevée et/ou une épaisseur de paroi plus faible, et/ou qu'au moins un renfoncement (14) est prévu dans chaque partie de paroi latérale (3, 4), les renfoncements (14) étant de préférence disposés de manière opposée.

8. Pessaire vaginal (1) selon l'une des revendications précédentes, **caractérisé en ce que** le volume de réception de la cavité (14) est compris entre 1 et 10 millilitres, de préférence entre 2 et 4 millilitres, et/ou **en ce qu'**une surface d'ouverture du renfoncement (14) est comprise entre 1 et 8 cm 2 , de préférence entre 2 et 3 cm 2 , et/ou **en ce qu'**une surface d'ouverture du renfoncement (14) présente, dans une vue latérale, un contour intérieur convexe et/ou ovale s'écartant de la forme circulaire.
